(19) Europäisches Patentamt
European Patent Office
Office européen des brevets

(11) EP 2 275 555 A2

(12) # EUROPÄISCHE PATENTANMELDUNG

(43) Veröffentlichungstag:
**19.01.2011 Patentblatt 2011/03**

(21) Anmeldenummer: **10011231.7**

(22) Anmeldetag: **03.01.2003**

(51) Int Cl.:
***C12N 15/55*** *(2006.01)* ***C12N 9/78*** *(2006.01)*
***C12N 15/63*** *(2006.01)* ***C12N 5/10*** *(2006.01)*
***C07K 16/40*** *(2006.01)* ***C12Q 1/34*** *(2006.01)*

(84) Benannte Vertragsstaaten:
**AT BE BG CH CY CZ DE DK EE ES FI FR GB GR HU IE IT LI LU MC NL PT SE SI SK TR**

(30) Priorität: **08.01.2002 DE 10200386**

(62) Dokumentnummer(n) der früheren Anmeldung(en) nach Art. 76 EPÜ:
**03000029.3 / 1 325 958**

(71) Anmelder: **GoEnzymes GmbH**
**79353 Bahlingen (DE)**

(72) Erfinder:
- **Gottschalk, Ellen-Marie**
  **37170 Nörten-Hardenberg (DE)**
- **Gottschalk, Gerhard**
  **37170 Nörten-Hardenberg (DE)**
- **Schmitz-Streit, Ruth Anne**
  **37085 Göttingen (DE)**
- **Thormann, Kai**
  **37120 Göttingen (DE)**

(74) Vertreter: **Schnappauf, Georg et al**
**Dr. Volker Vossius**
**Patent- und Rechtsanwaltskanzlei**
**Geibelstrasse 6**
**81679 München (DE)**

Bemerkungen:
Diese Anmeldung ist am 28-09-2010 als Teilanmeldung zu der unter INID-Code 62 erwähnten Anmeldung eingereicht worden.

(54) **Genetische Sequenz, die für Creatinin-Deiminase kodiert und deren Verwendung**

(57) Die vorliegende Erfindung betrifft genetische Sequenzen, die für Proteine oder Polypeptide mit Creatinin-Deiminase-Aktivität kodieren. Des weiteren betrifft die Erfindung die Verwendung dieser Proteine oder Polypeptide zur Bestimmung der Menge an Creatinin in einer Probe.

EP 2 275 555 A2

## Beschreibung

Die Synthese von Creatinin erfolgt im menschlichen Körper in Teilschritten, an denen mehrere Körperorgane beteiligt sind; vgl. Abb. 1 (NARAYAMAN, S., APPLETON, H.D. (1980). Clin. Chem. 26: 1119-1125).

In den Nieren entsteht aus Arginin und Glycin Ornithin und Guanidinacetat. Dieses gelangt über die Blutbahn zur Leber, wo aus Guanidinacetat Creatin synthetisiert wird, das über die Blutbahn in die Muskulatur gelangt. Im Muskel wird Creatin durch Creatinkinase (EC 2.7.3.2.) zum energiereichen Creatinphosphat phosphoryliert. Creatinphosphat dient im Muskel zur schnellen Überbrückung der Energieversorgung als Reservephosphat, das ATP kontinuierlich regeneriert. In der Erholungsphase erfolgt eine rasche Rephosphorylierung des Creatins zu Creatinphosphat und der Abbau des Creatinphosphats zu Creatinin unter Abspaltung von anorganischem Phosphat.

Creatinin wird in den Nieren glomerulär filtriert und ausgeschieden. Die Ausscheidung von Creatinin erfolgt in konstanter Beziehung zur Muskelmasse und zum Körpergewicht. Erhöhungen des Creatininspiegels im Plasma weisen bei normaler Muskelfunktion auf Nierenfunktionsstörungen hin.

Das endogen gebildete Creatinin wird in den Nieren bei normaler Plasmakonzentration weder reabsorbiert noch sezerniert. Es kann daher als Bezugsgröße für die Diagnostik renaler Störungen herangezogen werden. Da die Creatinin-Konzentration im Plasma isoliert betrachtet ohne eine diagnostische Sensitivität ist, erlaubt ihre alleinige Bestimmung keine ausreichende Beurteilung der Nierenfunktion bzw. der glomerulären Filtrationsrate. Eine für klinische Belange ausreichende quantitative Aussage über die glomeruläre Filtrationsrate wird durch die endogene Creatinin-Clearance erhalten, die eine exakte Ermittlung der Konzentrationen von Creatinin im Plasma und Urin zugrunde liegt. Indikation zur Durchführung einer endogenen Creatinin-Clearance ist das Erfassen einer eingeschränkten glomerulären Filtrationsrate bei Erstuntersuchungen, pathologischen Hambestandteilen, Hypertonie, akut und chronischen Nierenkranken, Hämodialysebehandlungen, Stoffwechselstörungen, Schwangerschaft und Medikation mit potentiellen nephrotoxischen Substanzen.

Zur Bestimmung der Creatininkonzentration im Plasma und Urin stehen prinzipiell chemische und enzymatische Nachweisverfahren zur Verfügung.

Bei dem vorherrschenden chemischen Verfahren (Methode nach Jaffé) wird Creatinin mit Pikrinsäure im alkalischen Milieu umgesetzt, wobei ein gelbrötlicher Farbstoff, ein Jankovskikomplex entsteht (JAFFÉ, M. (1886) Z. Physiol. Chem. 10: 391-400), der photometrisch bei einer Wellenlänge von 500-550 nm gemessen wird. Nachteil dieses Verfahrens ist jedoch eine geringe Spezifität; denn zahlreiche Nicht-Creatinin-Chromogene wie z. B. Bilirubin, Glukose, Ketonkörper, Acetoacetat und Pharmaka wie Cephalosporine und Metamizol bilden mit alkalischem Pikrat ein ähnliches Reaktionsprodukt wie Creatinin (SOLDIN, S.J., Henderson, L., Hill, J.G. (1978). Clin. Chem. 26: 286-290; KROLL, M.H., HAGENGRUBER, C., ELIN, R.J. (1985). J. Biol. Chem. 115: 333-341; SWAIN, R.R, BRIGGS, S.L. (1977). Clin. Chem. 23: 1340-1342; SAAH, A.J., KOCH, T.R. DRUSANO, G.L. (1982). JAMA 247: 205). In der Vergangenheit wurden verschiedenste Versuche unternommen, dieses Verfahren zu spezifizieren. Eine Modifikation bestand darin, Creatinin an Fullererde zu absorbieren, um so die wahre Creatininkonzentration zu ermitteln (KNOLL, E., STAMM, D. (1970). J. Clin. Chem. Clin. Biochem. 8: 582-587; KNOLL, E., WISSER, H. (1973). Z. Klin. Chem. Klin. Biochem. 11: 411). Zum anderen wurde in einigen Autoanalysem der "continous-flow-Generation" versucht, über eine Vordialyse dieses Verfahren vom Einfluss wesentlicher Störgrößen zu befreien (POPPER, H., MANDEL, E. MAYER, H. (1969). Biochem. Z. 291: 394; SCHEUERBRANDT, G., HELGER, R. (1969). Ärtztl. Lab. 15: 65).

Bei einem weiteren chemischen Verfahren wird Creatinin mit o-Nitrobenzaldehyd zu Methylguanidin abgebaut und mit Hilfe der Sahaguchi-Reaktion nachgewiesen (Van PILSUM, J.F., MARTIN, R.P., KITO, E. HESS, J. (1956). J. Biol. Chem. 222: 225-236).

Des weiteren ist ein chemisches Verfahren bekannt, bei dem 3,5-Dinitrobenzoesäure bzw. 3,5-Dinitrobenzoylchlorid eingesetzt wird, das mit Creatinin einen purpurroten Farbkomplex bildet (LANGLEY, W.D., EVENS, M. (1936). J. Biol. Chem. 115: 333-341; BENEDICT, S.R., BEHRE, J.A. (1936). J. Biol. Chem. 114: 515-532; SIROTA, J.H, BALDWIN, D.S., VILLAREAL, H. (1950). J. Clin. Invest. 29: 187-192).

Keines dieser chemischen Verfahren weist jedoch eine gegenüber der Jaffé-Methode verbesserte Spezifität auf.

Prinzipiell stehen drei unterschiedliche enzymatische Verfahren zur Bestimmung der Creatininkonzentration im Plasma und Urin zur Verfügung. Bei zwei dieser Verfahren wird Creatinin im ersten Reaktionsschritt mittels Creatininase (EC 3.5.2.10) zu Creatin umgesetzt.

Bei einem Verfahren liefert die Umsetzung von Creatin zu Creatinphosphat in Gegenwart von Creatinkinase (EC 2.7.3.2.) ADP, welches mit PEP von Pyruvatkinase (EC 2.7.1.40) zu Pyruvat und ATP umgesetzt wird; vgl. Abb. 2. Das Pyruvat wird in der Indikatorreaktion in Gegenwart von Laktatdehydrogenase (EC 1.1.1.27) zu Laktat umgesetzt. Hierbei wird der Verbrauch von NADH durch eine Extinktionsabnahme bei 340 nm gemessen, die mit der Creatininkonzentration in der untersuchten Probe in Beziehung steht.

In einem weiteren Verfahren wird das durch die enzymatische Umsetzung von Creatinin mit Creatininase (EC 3.5.2.10.) erhaltene Creatin durch 2 Hilfsreaktionen mittels Creatinase (EC 3.5.3.3.) und Sarcosinoxidase (EC 1.5.3.1.) zu Glycin, Formaldehyd und $H_2O_2$ abgebaut; vgl. Abb. 3. Die Menge an gebildetem $H_2O_2$ wird in der Indikatorreaktion, einer modifizierten Trinderreaktion PAP, mittels Peroxidase (EC 1.11.1.7.) anhand der Extinktionszunahme bei 510 oder 546 nm bestimmt (GUDER, W.G., HOFFMANN, G.E., POPPE, W.A., SIEDEL, J., PRICE, C.P. (1986). J. Chem. Clin. Biochem. 24: 889-902).

Ein weiteres Verfahren beruht auf der durch Creatinin-Deiminase (EC 3.5.4.21) katalysierten Spaltung von Creatinin zu N-Methylhydantoin und Ammoniak (Messreaktion, SZULMAJSTER, J. (1958). J. Bacteriol. 75: 633-639). Die Menge an gebildetem Ammoniak kann in einer Mehrschichtfilmtechnik mittels Indikator bestimmt werden (Indikatorreaktion, SHIREY, T.L. (1983). Clin. Biochem. 16: 147-152). Alternativ kann Ammoniak mit $\alpha$-Ketoglutarat und NADPH/$H^{\oplus}$ mittels Glutamat-Dehydrogenase zu Glutamat umgesetzt und photometrisch die Extinktionsabnahme bei 340 bzw. 365 nm gemessen werden (LIM, F. (1974). Clin. Chem. 20: 871; TANGANELLI, E., PRINCIPE, L., BASSI, D., CAMBIAGHI, S., MURADOR, E. (1982). Clin. Chem. 28: 1461-1464); vgl. Abb. 4.

Die oben beschriebenen enzymatischen Verfahren unterliegen nicht den zahlreichen Störfaktoren der heute noch weitverbreiteten Jaffé-Methode. Allerdings scheiterte das von Tanganelli vorgeschlagene Verfahren an der unzureichenden Substratspezifität der in der Messreaktion eingesetzten Creatinin-Deimininase, die neben Creatinin auch Cytosin und verwandte Derivate umsetzte. Diese positiven Interferenzen führten bei Anwendung des enzymatischen Tanganellischen Testes zu falsch hohen Creatininwerten - einer Pseudohypercreatininämie.

Es ist daher die Aufgabe der vorliegenden Erfindung, Mittel und Verfahren bereitzustellen, die eine Bestimmung der Menge an Creatinin in einer Probe mit hinreichender Genauigkeit erlauben, wobei Interferenzen mit anderen Substanzen, insbesondere Cytosin, bei der Messung möglichst vermieden werden sollen. Diese Mittel und Verfahren sollen insbesondere eine kostengünstige Untersuchung großer Probenserien bei angemessener Geschwindigkeit ermöglichen.

Diese Aufgabe wird erfindungsgemäß durch den Gegenstand der Patentansprüche gelöst.

Die Erfindung betrifft eine Nukleinsäuresequenz, die für eine Protein oder Polypeptid mit der Aktivität einer Creatinin-Deiminase kodiert. In einer Ausführungsform betrifft die Erfindung die in SEQ ID NO:1 gezeigte Nukleinsäuresequenz oder einen Teil oder ein Derivat hiervon.

Der Begriff "Nukleinsäuresequenz, die für ein Protein oder Polypeptid mit der Aktivität einer Creatinin-Deiminase kodiert" wird hier in der allgemeinsten Form gebraucht und umfasst jede Abfolge von Nukleotidbasen, die direkt oder über eine komplementäre Reihe von Nukleotidbasen eine Aminosäuresequenz eines Proteins oder Polypeptids mit der Aktivität einer Creatinin-Deiminase bestimmt.

Mit dem Begriff "Aktivität einer Creatinin-Deiminase" ist jede enzymatische Aktivität gemeint, die Creatinin zu N-Methylhydantoin und Ammoniak umsetzt.

Mit "Derivat" einer Nukleinsäuresequenz ist gemeint, dass einzelne oder multiple Nukleotidsubstitution, -deletion und/oder -addition in der Nukleinsäuresequenz vorliegen. Weiterhin umfasst der Begriff "Derivat" auch eine chemische Derivatisierung einer Nukleinsäuresequenz an einer Nukleotidbase, am Zucker oder am Phosphat. Der Begriff "Derivat" umfasst auch Nukleinsäuresequenzen, die nicht in der Natur vorkommende Nukleotide und Nukleotidanaloga enthalten.

Die vorliegende Erfindung betrifft auch Nukleinsäuresequenzen, die unter niedrig-, besser unter mittel- und am besten unter hoch-stringenten Bedingungen mit der in SEQ ID NO:1 dargestellten Sequenz oder einem Teil oder einem Bereich davon hybridisieren. In einer weiteren Ausführungsform betrifft die Erfindung eine Nukleinsäuresequenz, die mindestens 50%, vorzugsweise mindestens 60%, besser mindestens 70% und noch besser mindestens 80% Homologie zu der in SEQ ID NO:1 gezeigten Nukleinsäuresequenz oder einem oder mehreren Bereichen davon aufweist. In einer besonders bevorzugten Ausführungsform weist die Nukleinsäuresequenz mindestens 90%, insbesondere mindestens 95% oder mindestens 98% Homologie zu einem oder mehreren Bereichen, vorzugsweise zu dem vollständigen Bereich der in SEQ ID NO:1 dargestellten Nukleinsäuresequenz auf. In einer weiteren Ausführungsform betrifft die Erfindung eine Nukleinsäuresequenz, die bezogen auf eine Nukleinsäuresequenz gemäß der vorhergehenden Ausführungsformen degeneriert ist. Vorzugsweise kodiert die erfindungsgemäße Nukleinsäuresequenz für ein Protein oder Polypeptid, das Cytosin nicht deaminiert.

Die erfindungsgemäße Nukleinsäuresequenz ist vorzugsweise Desoxyribonukleinsäure (DNA) oder Ribonukleinsäure (RNA). Sie kann aus dem natürlichen Umfeld isoliert oder *in vitro* hergestellt oder synthetisiert werden. Die erfindungsgemäßen Nukleinsäuresequenzen umfassen genomische DNA, cDNA, rekombinant hergestellte und chemisch synthetisierte Moleküle. In einer besonders bevorzugten Ausführungsform ist die erfindungsgemäße Nukleinsäuresequenz aus *Tissierella creatinini* abgeleitet. In einer weiteren bevorzugten Ausführungsform ist die erfindungsgemäße Nukleinsäuresequenz eine rekombinante Nukleinsäuresequenz. Die erfindungsgemäße Nukleinsäuresequenz kann als einzelsträngiges oder doppelsträngiges und lineares oder kovalent kreisförmig geschlossenes Molekül vorliegen.

Des weiteren betrifft die Erfindung eine Nukleinsäuresequenz, die zu der erfindungsgemäßen Nukleinsäuresequenz, die für eine Protein oder Polypeptid mit der Aktivität einer Creatinin-Deiminase kodiert, komplementär ist. Eine Nukleinsäuresequenz ist dann zu einer anderen Nukleinsäuresequenz "komplementär", wenn die beiden Sequenzen miteinander hybridisieren und ein stabiles Duplex eingehen können, wobei die Hybridisierung vorzugsweise unter Bedingungen erfolgt, die eine spezifische Hybridisierung zwischen Polynukleotiden erlauben (stringente Bedingungen).

Die erfindungsgemäßen Nukleinsäuresequenzen können alleine oder in Kombination mit anderen Nukleinsäuresequenzen, insbesondere heterologen Nukleinsäuresequenzen, vorliegen. In bevorzugten Ausführungsformen liegt die erfindungsgemäße Nukleinsäuresequenz in Verbindung mit Expressionskontrollsequenzen vor, die in Bezug zu der erfindungsgemäßen Nukleinsäuresequenz homolog oder heterolog sein können. Der Begriff "Expressionskontrollsequenz" umfasst erfindungsgemäß Promotoren, Enhancer und andere Kontrollelemente, die die Expression eines Gens steuern. In bestimmten erfindungsgemäßen Ausführungsformen sind die Expressionskontrollsequenzen regulierbar. Des weiteren kann die erfindungsgemäße Nukleinsäuresequenz in Verbindung mit einer Nukleinsäuresequenz vorliegen, die für ein Polypeptid kodiert, das eine Sekretion des durch die erfindungsgemäße Nukleinsäuresequenz kodierten Proteins oder Polypeptids aus einer Wirtszelle steuert.

Somit wird erfindungsgemäß ein rekombinantes DNA-Molekül bereitgestellt, das die vorstehend erläuterte Nukleinsäuresequenz, die für ein Protein oder Polypeptid mit der Aktivität einer Creatinin-Deiminase kodiert, oder eine komplementäre Nukleinsäuresequenz umfasst. In einer bevorzugten Ausführungsform ist das rekombinante DNA-Molekül ein Vektor oder ein Plasmid, gegebenenfalls mit einem Promotor, der die Expression der erfindungsgemäßen Nukleinsäuresequenz steuert. Der Begriff "Vektor" wird dabei in seiner allgemeinsten Bedeutung verwendet und umfasst jegliche intermediären Vehikel für eine Nukleinsäure, die es z.B. ermöglichen die Nukleinsäure in prokaryotische und/ oder in eukaryotische Zellen einzubringen und gegebenenfalls in ein Genom zu integrieren. Solche Vektoren werden vorzugsweise in der Zelle repliziert und/oder exprimiert. Ein intermediäres Vehikel kann z.B. für den Gebrauch bei der Elektroporation, beim Mikroprojektilbeschuss, beim Transfer mit Hilfe von Agrobakterien oder bei der Insertion über DNA- oder RNA-Viren angepasst sein.

Die Erfindung betrifft auch Wirtszellen, die eine erfindungsgemäße Nukleinsäuresequenz enthalten. Der Begriff "Wirtszelle" betrifft jede Zelle, die mit einer exogenen Nukleinsäuresequenz transformierbar oder transfizierbar ist. Wirtszellen können prokaryotische oder eukaryotische Zellen sein, insbesondere Hefezellen, Insektenzellen, Pflanzenzellen und Säugerzellen. Bevorzugte prokaryotische Zellen sind *Escherichia coli* oder *Bacillus subtilis.* Die erfindungsgemäße Nukleinsäuresequenz kann in der Wirtszelle in einer einzigen oder in mehreren Kopien vorliegen und wird in einer Ausführungsform in der Wirtszelle exprimiert. Der Begriff "Expression" wird erfindungsgemäß in seiner allgemeinsten Bedeutung verwendet und umfasst die Produktion von RNA oder von RNA und Protein. Er umfasst auch eine teilweise Expression von Nukleinsäuresequenzen. Des weiteren kann die Expression transient oder stabil erfolgen.

In einer weiteren Ausführungsform betrifft die Erfindung Oligonukleotide, die mit einer erfindungsgemäßen Nukleinsäuresequenz hybridisieren und als genetische Sonden oder als "Antisense"-Moleküle verwendet werden können. Nukleinsäuremoleküle in der Form von Oligonukleotid-Primern oder kompetenten Proben, die mit einer erfindungsgemäßen Nukleinsäuresequenz oder Teilen davon, insbesondere mit der in SEQ ID NO:1 dargestellten Nukleinsäuresequenz hybridisieren, können zum Auffinden von Nukleinsäuresequenzen verwendet werden, die zu der erfindungsgemäßen Nukleinsäuresequenz homolog sind und für ein Protein oder Polypeptid mit der Aktivität einer Creatinin-Deiminase kodieren. PCR-Amplifikation, Southern- und Northern-Hybridisierung können zum Auffinden homologer Nukleinsäuresequenzen eingesetzt werden. Die Hybridisierung kann unter niedrig-, besser unter mittel- und am besten unter hoch-stringenten Bedingungen erfolgen. Der Begriff "stringente Bedingungen" betrifft erfindungsgemäß Bedingungen, die eine spezifische Hybridisierung zwischen Polynukleotiden erlauben.

"Antisense"-Moleküle können zur Regulierung, insbesondere der Reduktion der Expression einer erfindungsgemäßen Nukleinsäuresequenz verwendet werden. Ein "Antisense-Molekül" umfasst erfindungsgemäß auch ein Konstrukt, das eine erfindungsgemäße Nukleinsäuresequenz oder einen Teil davon in reverser Orientierung in Bezug auf einen Promotor enthält. Ein Antisense-Transkript der erfindungsgemäßen Nukleinsäuresequenz oder ein Teil davon kann eine Duplex mit der natürlich vorkommenden mRNA, die das Enzym spezifiziert, eingehen und so eine Akkumulation von oder die Translation der mRNA in das aktive Enzym verhindern. Eine weitere Möglichkeit ist die Verwendung von Ribozymen zur Inaktivierung der erfindungsgemäßen Nukleinsäuresequenzen. Bevorzugte erfindungsgemäße Oligonukleotide weisen eine Länge von 6 bis 50 Nukleotiden, insbesondere 10 bis 30 Nukleotiden und am besten 15 bis 20 Nukleotiden auf und sind vorzugsweise vollständig zu der erfindungsgemäßen Nukleinsäuresequenz oder einem Teil davon komplementär.

Die Erfindung betrifft weiterhin ein Protein oder Polypeptid mit der Aktivität einer Creatinin-Deiminase. Das Protein oder Polypeptid wird vorzugsweise durch eine erfindungsgemäße Nukleinsäuresequenz kodiert. In einer weiteren bevorzugten Ausführungsform betrifft die Erfindung ein Protein oder Polypeptid, das die in SEQ ID NO:2 gezeigte Aminosäuresequenz oder einen Teil oder ein Derivat davon umfasst.

"Derivate" eines Proteins oder Polypeptids oder einer Aminosäuresequenz im Sinne dieser Erfindung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise ist das erfindungsgemäße Protein oder Polypeptid mindestens 50%, vorzugsweise mindestens 60%, besser mindestens 70% und noch besser mindestens 80% zu der in SEQ ID NO:2 gezeigten Sequenz homolog. In einer besonders bevorzugten Ausführungsform beträgt der Grad an Homologie mindestens 90%, insbesondere mindestens 95% oder mindestens 98%.

Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften ersetzt, wie Hydrophobizität, Hydrophilizität, Elektronegativität, Volumen der Seitenkette und ähnliches (konservative Substitution). Konservative Substitutionen betreffen beispielsweise den Austausch einer Aminosäure durch eine andere, nachstehend in derselben Gruppe wie die substituierte Aminosäure aufgeführte Aminosäure:

1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch "Solid Phase Synthesis" (MERRIFIELD, R.B., STEWART J.M. (1965). Nature 207: 522-523) und ähnliche Verfahren oder durch rekombinante DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen ist z.B. in Sambrook et. al. (SAMBROOK, J., FRITSCH, E. F., MANIATIS, T. (1989). Cold Spring Harbour Laboratory) ausführlich beschrieben.

"Derivate" der erfindungsgemäßen Proteine oder Polypeptide umfassen auch einzelne oder multiple Substitutionen, Deletionen und/oder Additionen jeglicher Moleküle, die mit dem Enzym assoziiert sind, wie Kohlenhydrate, Lipide und/oder Proteine oder Polypeptide.

Ferner erstreckt sich der Begriff "Derivat" auch auf alle funktionellen chemischen Äquivalente der erfindungsgemäßen Proteine oder Polypeptide.

In einem weiteren Aspekt betrifft die Erfindung ein Verfahren zur Herstellung eines Proteins oder Polypeptides mit Creatinin-Deiminaseaktivität, wobei eine erfindungsgemäße Nukleinsäuresequenz, die für ein Protein oder Polypeptid mit der Aktivität einer Creatinin-Deiminase kodiert, in einer Wirtszelle exprimiert und das Protein oder Polypeptid isoliert wird. Vorzugsweise erfolgt die Isolierung des Proteins oder Polypeptids durch Ammoniumsulfatfällung, Chromatographie an Phenylsepharose und DEAE-SepharoseCL6B.

In einem weiteren Aspekt betrifft die Erfindung ein Verfahren zur Bestimmung des Creatinin-Gehalts in einer Probe, vorzugsweise in einer Körperflüssigkeit, insbesondere Plasma, Serum oder Urin, durch Verwendung des erfindungsgemäßen Proteins oder Polypeptids mit der Aktivität einer Creatinin-Deiminase. Eine derartige Bestimmung des Creatinin-Gehalts kann Aufschluss über die glomeruläre Filtrationsrate, d.h. die in einer Zeiteinheit filtrierte Flüssigkeitsmenge, der Niere geben. Die Bestimmung von Creatinin in Körperflüssigkeiten kann ferner zur Diagnose von Muskelerkrankungen oder verschiedenen Nierenerkrankungen wie Nephritis oder Niereninsuffizienz dienen.

Vorzugsweise wird die Probe mit einer erfindungsgemäßen Creatinine-Deiminase behandelt und die Menge des gebildeten Ammoniaks bestimmt. In einer bevorzugten Ausführungsform erfolgt die Bestimmung des Ammoniaks durch Behandlung mit Glutamatdehydrogenase in Gegenwart von $\alpha$-Ketoglutarat und NADH (bzw. NADPH) und Messung des Verbrauchs an NADH (bzw. NADPH), vorzugsweise durch photometrische Messung bei 340 bzw. 365 nm.

Dieses erfindungsgemäße Verfahren ermöglicht die Bestimmung von Creatinin bei Einsatz von nur zwei Enzymen, die in einer Mess- bzw. Indikatorreaktion eingesetzt werden, wobei keine komplizierten Hilfsreaktionen erforderlich sind. Wie in Tabelle 1 gezeigt ist, weist die erfindungsgemäße Creatinin-Deiminase eine außerordentlich hohe Spezifität für sein Substrat Creatinin auf. Das Enzym zeigt insbesondere mit Cytosin bis zu einer Konzentration von 2000 mg/ml keine Interferenz in der Reaktion. Somit ermöglicht die erfindungsgemäße Creatinin-Deiminase eine Bestimmung von Creatinin auch in Gegenwart anderer Substrate, insbesondere Cytosin ohne das Auftreten einer Interferenz, die das Messergebnis verfälschen würde.

Tabelle 1: Interferenzstudien mit der gereinigten heterolog exprimierten Creatinin-Deiminase aus *Tissierella creatinini* mit verschiedenen in Seren relevanten Substanzen. Diese wurden zum Standardtestansatz zugegeben und jeweils die Aktivität der Creatinin-Deiminase bestimmt. Bei den aufgeführten Substanzen traten in den angegebenen Konzentrationen keine Interferenzen mit dem Enzymtest auf.

| **Verbindung/Lösung** | **Keine Interferenzen bis zu einer Konzentration von (mg/l)** |
|---|---|
| Acetoacetat/$H_2O$ dest. | 7.8 |
| Aceton/ $H_2O$ dest. | 2000 |
| Ascorbinsäure/ $H_2O$ dest. | 200 |
| Bilirubin | 210 |
| Captopril | 83 |
| Ceficitin/ $H_2O$ dest. | 860 |
| Creatin/ $H_2O$ dest. | 2000 |
| Cytosin | 2000 |
| Epinephrin/ $H_2O$ dest. | 0,05 |
| Etomedac | 666 |
| Fluoroblastin | 33 |
| 5-Fluorocytosin $H_2O$ dest. (Ancotil) | 1000 |
| Fuoresemid | 4433 |
| Glukose | 1100 |
| Hämoglobin (hämolysiertes Serum) | kein |
| Histidin/*aqua bidest* | 2250 |
| IgM | 20.000 |
| $\alpha$-Methyl-Dopa/ $H_2O$ dest. | 107 |
| Neogama D novo | 666 |
| Nitrofurantoin/ $H_2O$ dest. | 200 |
| Norfenefrin/ $H_2O$ dest. | 1,5 |
| Oxalsäure/ $H_2O$ dest. | 400 |
| Predni H Tablinen (Prednisolon) | 278 |
| Pyruvat/ $H_2O$ dest. | 5,9 |
| Salazosulfapyridin/Methanol | 550 |
| Sulfatmethoxazol/Aceton | 500 |
| Trimethoprim/Methanol | 46 |
| Vancomycin | 288 |

Die vorliegende Erfindung betrifft auch einen Kit zur Bestimmung der Creatinin-Konzentration. Der erfindungsgemäße Kit kann eine erfindungsgemäße Nukleinsäuresequenz, die für ein Protein oder Polypeptid mit Creatinin-Deiminase-Aktivität kodiert, eine Wirtszelle, die eine derartige Nukleinsäure enthält oder ein erfindungsgemäßes Protein oder Polypeptid mit Creatinin-Deiminase-Aktivität umfassen. Weiterhin können in dem Kit Reagenzien enthalten sein, die der Messung der Creatininmenge in einer Probe dienen und die in dieser Erfindung beschrieben sind.

Die vorliegende Erfindung wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich, die ebenfalls erfindungsgemäß umfasst sind.

**Figuren:**

<u>Abb. 1:</u> **Biosynthese von Creatin und Creatinin im menschlichen Körper.** Die Synthese erfolgt in mehreren Schritten in verschiedenen Körperorganen.

<u>Abb. 2:</u> **Enzymatische Bestimmung von Creatinin mit Creatininase, Creatin-Kinase, Pyruvat-Kinase und**

**Laktatdehydrogenase.** Creatinin wird katalysiert durch Creatininase und Creatin-Kinase gekoppelt mit Pyruvatbildung. Das Pyruvat wird in der Indikatorreaktion in Gegenwart von Laktatdehydrogenase zu Laktat umgesetzt und dabei eine Extinktionsabnahme bei 340 nm von NADH gemessen.

**Abb. 3: Enzymatische Bestimmung von Creatinin mit Creatininase, Creatinase, Sarcosinoxidase und Peroxidase.** Creatinin wird katalysiert durch Creatininase, Creatinase und Sarcosinoxidase zu Glycin, Formaldehyd und $H_2O_2$ abgebaut. Das $H_2O_2$ wird in der Indikatorreaktion mittels Peroxidase anhand der Extinktionszunahme bei 510 oder 546 nm bestimmt.

**Abb. 4: Enzymatische Bestimmung von Creatinin mit Creatinin-Deiminase und Glutamatdehydrogenase.** Creatinin wird katalysiert durch Creatinin-Deiminase zu N-Methylhydantoin und Ammoniak abgebaut. Der Ammoniak wird mit α-Ketoglutarat und NADH mittels Glutamat-Dehydrogenase zu Glutamat umgesetzt und photometrisch die Extinktionsabnahme durch Verbrauch an NADH bei 340 bzw. 365 nm gemessen.

**Abb. 5: Schematische Darstellung des pBluescriptSK$^+$-Vektors.** Gezeigt sind einige Restriktionsschnittstellen. Abkürzungen: *f1* ori: Phage *f1* Replikationsursprung; Ap: Ampicillinresistenz; ORI: Replikationsursprung für *E. coli*; *LacZ*: α-Komplementationsstück der β-Galaktosidase. Größenangaben in kbp.

**Abb. 6: Schematische Darstellung des rekombinanten Vektors pKT1**. Gezeigt sind das klonierte Fragment mit den flankierenden *Hind*III-Schnittstellen. Abkürzungen: *cdi*: bezeichnet die Lage und Ableserichtung des Gens der Creatinin-Deiminase-Untereinheit. Die Größenangaben bedeuten kbp.

**Abb. 7: DNA-Sequenz der Creatinin-Deiminase Untereinheit (*cdi*) von *T. creatinini.*** Die abgeleitete Aminosäuresequenz ist unterhalb der DNA-Sequenz im Ein-Buchstaben-Code aufgeführt.

**Abb. 8: Gelelektrophorese unter nativen Bedingungen zum Expressionsnachweis der Creatinin-Deiminase von *T. creatinini* nach heterologer Expression in *E. coli.*** Die Größe der mit Pfeilen gekennzeichneten Banden ist in kDa angegeben. Spur 1: Proteinstandard (Thyroglobulin, Ferritin, Katalase, Laktat-Dehydrogenase, Albumin); Spur 2: Rohextrakt von *Tissierella creatinini* (ca. 25 μg); Spur 3 und 5: aufgereinigte Creatinin-Deiminase (5 μg); Spur 4: Rohextrakt von *E. coli* DH5α/pKT1 (ca. 15 μg); Spur 6: Rohextrakt von *E. coli DH5α*/pBluescript (ca. 15 μg).

**Abb. 9: Analyse der aufgereinigten, rekombinant exprimierten Creatinin-Deiminase von *T. creatinini.*** Es erfolgte eine **Auftrennung** durch PhastGel-Elektrophorese mit einem Polyacrylamid-Gradienten von 8-25%. Spur 1, 6: Proteinstandard; Spur 2,3: natives aufgereinigtes Enzym; Spur 4,5: aufgereinigte, mit SDS denaturierte Creatinin-Deiminase (1 μg).

**Abb. 10: Westernblot-Analyse der aufgereinigten, rekombinant exprimierten Creatinin-Deiminase von *T. creatinini..*** Aufgereinigte Creatinin-Deiminase von *T. creatinini* und Gesamtzellextrakt von *T. creatinini* wurden gelelektrophoretisch (12,8 % PAGE) unter denaturierenden Bedingungen aufgetrennt, die Proteine auf eine Nitrozellulosemembran transferiert und Creatinin-Deiminase mittels eines polyklonalen Antikörpers immunologisch nachgewiesen. Spur 1-3: rekombinantes, aufgereinigtes Enzym; Spur 5 bis 7: Zellextrakt *aus T. creatinini.*

**Abb. 11: Substratabhängigkeit der aufgereinigten, heterolog exprimierten Creatinin-Deiminase aus *T. creatinini.*** Die Enzymaktivität ist in Abhängigkeit von der Creatininkonzentration aufgetragen. Eingefügte Abbildung: Doppeltreziproke Auftragung nach Lineweaver-Burk. In den Aktivitätstest wurde jeweils 0,06 μg gereinigtes Enzym eingesetzt.

**Abb. 12: Creatinin-Bestimmung unter Verwendung der Creatinin-Deiminase von *T. creatinini* im gekoppelten optischen Test auf einem Hitachi 717-Gerät.** Die Bestimmungsmethode ist mit der enzymatischen Creatinin-Bestimmung von Roche-Diagnostik (Testprinzip vgl. Abb. 3) verglichen. Das verwendete Hitachi 717-Gerät wurde mittels käuflicher Standards kalibriert. Y-Achse: Gekoppelter optischer Test mit der Creatinin-Deiminase, X-Achse: Enzymatische Creatinin-Bestimmung mittels des Roche-Diagnostik-Kits.

**Beispiele:**

**Beispiel 1: Klonierung des Creatinin-Deiminase-Gens (*cdi*-Gen) aus *T. creatinini***

Reinigung der Creatinin-Deiminase aus *Tissierella creatinini*

Die Kultivierung von *Tissierella creatinini* erfolgte in Hungate-Röhrchen (Bellco Glass Inc., Vineland, USA) stehend bei 37 °C in einem anaeroben Medium, das gemäß der von Bryant (BRYANT, M. P. (1972). Am. J. Clin. Nutr. 25: 1324-1328) modifizierten Hungate-Technik (HUNGATE, R. E. (1969). In: NORRIS, J. R. RIBBONS, D. W. (Hrsg.) Methods in Microbiology 3B: 117-132. Academic press, London) hergestellt wurde. Nach Einstellen des pH-Wertes wurde das Medium mit $N_2/CO_2$ (80/20 % (v/v)) durchgast, verschlossen und dann bei 121°C 20-30 min autoklaviert. Vor dem Animpfen erfolgte die Zugabe des getrennt autoklavierten Reduktionsmittels (L-Cystein, 5 % (w/v)).

Der Aufschluss der Zellen erfolgte mittels einer French-Press-Zelle (SLM Instruments Company, Urbana, USA) bei einem Druck von 62 bis 103 Mpa. Zelltrümmer und nicht aufgeschlossene Zellen wurden durch Zentrifugation (10000-12000× g, 10 min, 4 °C) entfernt. Der zellfreie Überstand ergab den Rohextrakt.

Die Creatinin-Deiminase von *Tissierella creatinini* (GAUGLITZ, U. (1988). Dissertation Universität Göttingen) wurde aus 15 g *T. creatinini* Zellen wie beschrieben zur apparenten Homogenität gereinigt (GOTTSCHALK, E. M., HIPPE, H., PATZKE, F. (1991). Clin. Chim. Acta 204: 223-238).

Sequenzierung von internen Peptiden der Creatinin-Deiminase

Die gereinigte Creatinine-Deiminase wurde mit der Endoproteinase Glu-C von *Staphylococcus aureus* V8 nach der Methode von Cleveland et al. (CLEVELAND, D. W., FISCHER, S. G., KIRSCHNER, M. W., LAEMMLI, U. K. (1977). J. Biol. Chem. 252: 1102-1106) im SDS-Gel gespalten, und die Spaltfragmente wurden auf eine PVDF-Membran (Immobilon-P Transfer-Membrane, Fa. Millipore Corporation, USA) unter Verwendung eines Semi-Dry Fast-Blot Gerätes (Multiphor II Nova Blot, Fa. Pharmacia LKB GmbH, Freiburg) übertragen. Der N-terminale Bereich der Creatinin-Deiminase und eines internen Peptids (28 kDa) wurden mittels eines Procise 491 Protein-Sequencers (Applied Biosystems, USA) sequenziert.

Isolierung chromosomaler DNA aus *T. creatinini*

Isolierung chromosomaler DNA aus *T. creatinini* erfolgte nach einer modifizierten Methode für DNA Isolierung aus Clostridien nach Bertram und Dürre (BERTRAM, J. DÜRRE, P. (1989). Arch. Microbiol. 151: 551-557).

Herstellung einer spezifischen Sonde gegen das Creatinin-Deiminase-kodierende Gen (*cdi-Gen) und Hybridisierung*

Aus den erhaltenen N-terminalen Aminosäuresequenzen der N-terminalen Domäne der Creatinin-Deiminase und des internen Peptids wurden heterologe Oligonukleotide abgeleitet. Unter Verwendung dieser Oligonukleotide und chromosomaler DNA aus *T. creatinini* als Matrize wurde in einer Polymerase-Ketten-Reaktion (PCR) eine 600 Basenpaare lange, spezifische Sonde des *cdi*-Gens gewonnen. Diese Sonde wurde mit dem *Nonradioactive-DNA-Labeling*-Kit von Boehringer Mannheim (Mannheim) markiert und in Southern-Hybridisierungen eingesetzt. In Southern-Hybridisierungen, die nach dem Standardprotokoll von Sambrock et al. 1989 (supra) bzw. Ausubel et al. 1987 (AUSUBEL, F., BRENT, R., KINGSTON, R.E., MOORE, D.D., SEIDMANN, J.G., SMITH, J.A. STRUHL, K. (1987). John Wiley and Sons, New York) durchgeführt wurden, wurde ein einzelnes 4,5 kbp großes *Hind*III-DNA Fragment identifiziert, das spezifisch mit der 600 bp-Sonde reagierte und somit das Creatinin-Deiminase-kodierende *cdi*-Gen beinhaltete.

Das *cdi*-Gen aus *T. creatinini* wurde mittels einer "Shotgun-Klonierung" in den Vektor $pKS^+$ (vgl. Abb. 5) kloniert. Hierzu wurde *Hind*III-restriktionsfragmentierte chromosomale *T. creatinini* DNA in den mit *Hind*III linearisierten pBluescript $SK^+$ ($pSK^+$)-Vektor (Stratagene, Heidelberg; $Ap^r$, *lac POZ'*) ligiert und *in Escherichia coli* DH5α (HANAHAN, D. (1983). J. Mol. Biol. 166: 557-580; Genotyp: F-, *lacZDM15, rec*A1*, hsdR17, supE44,* Δ(*lacZYA, argF*) transformiert. Die Kultivierung von *E. coli* erfolgte wie beschrieben in Luria Bertani (LB) Medium mit den entsprechenden Medienzusätzen (Sambrock et al., 1989, supra).

Die erhaltenen Klone wurden durch Koloniehybridisierung mit der markierten 600 bp Sonde auf das Vorhandensein des *cdi*-Gen durchmustert. Dabei wurde die Koloniehybridisierung wie bei Sambrock et al. 1989 (supra) beschrieben durchgeführt. Die Detektion der DIGmarkierten Sonde erfolgte nach den Angaben des Herstellers (Boehringer, Mannheim) unter Verwendung der CSPD-Detektionslösung. Es wurde ein positiver Klon identifiziert, der ein 4,5 kbp großes Insert aufwies. Dieses rekombinante Plasmid wurde als pKT1 (4,5 kb *Hind*III DNA Fragment von *T. creatinini* in $pSK^+$) bezeichnet. Mittels Restriktionskartierung des 4,5 kbp-Fragments wurde die Lage des vollständigen *cdi*-Gens im Fragment bestimmt (vgl. Abb. 6).

### Beispiel 2: Sequenzierung und Analyse des *cdi*-Gens aus *T. creatintini*

Die Sequenz des *cdi*-Gens auf dem 4,5 kbp Insert von pKT1 wurde auf beiden Strängen durch die Methode des "Primer walking" bestimmt (STRAUSS, E.C., KOBORI, J.A., SIU, G., HOOD, L.E. (1986). Anal. Biochem. 154: 353-360). Dabei erfolgten die DNA-Sequenzierungen unter Verwendung des Automated Laser Flourescent Sequencer (ALF) plus einem PC mit der dazugehörigen Steuersoftware "ALF-Manager v. 2.6" (Pharmacia LKB, Freiburg). Für die Sequenzreaktionen wurde der "AutoRead Sequencing Kit" (Pharmacia LKB, Freiburg) verwendet. Sequenzierungen erfolgten mit den mitgelieferten fluoreszensmarkierten universellen Primern des Kits bzw. mit aus der Sequenz abgeleiteten fluoreszensmarkierten Primern (MWG-Biotech GmbH (Ebersberg)).

Die Analyse der Sequenzdaten erfolgte mit dem Programm DNA STRIDER Version 1.2 (MARCK, C. (1988). Nucl. Acids Res. 16: 1829-1836) auf einem Macintosh Computer (Fa. Apple Computer, Cupertino, USA). Weitergehende Sequenzanalysen wurden mit Hilfe des "Wisconsin GCG Sequence Analysis Software Package" Version 8 (Genetics Computer Group, University of Wisconsin Biotechnologie Center, Madison, USA) auf einem Großrechner mit dem Betriebssystem UNIX durchgeführt. Für Sequenzvergleiche wurden die Gen- und Proteindatenbanken EMBL, GenBank und SwissProt herangezogen. Das *cdi*-Gen wies eine Länge von 1218 bp auf, die für 406 Aminosäuren kodieren. Daraus errechnet sich ein Molekulargewicht von 47,5 kDa, was gut mit der molekularen Masse (47,5 kDa) übereinstimmt, die mittels gelelektrophoretischer Auftrennung des gereinigten Proteins unter denaturierenden Bedingungen bestimmt wurde. Die vollständige Sequenz des *cdi*-Gens mit der abgeleiteten Aminosäuresequenz ist in Abb. 7 dargestellt.

Die abgeleitete Aminosäuresequenz der Creatinin-Deiminase von *T. creatinini* wies hohe Ähnlichkeiten zu der Cytosin-Deaminase (*codA*) von *E. coli* auf, welche in der Lage ist, cometabolisch Creatinin zu deaminieren. Stromabwärts des *cdi*-Gens befindet sich der N-terminale Bereich eines weiteren offenen Leserahmens (*orf*), dessen abgeleitete Aminosäuresequenz hohe Homologien zur N-Carbamoylsarkosin-Amidohydrolase von *Arthrobacter sp.* aufweist (vgl. Abb. 6). Damit scheint dieser *orf* für den aminoterminalen Teil der N-Carbamoylsarkosin-Amidohydrolase von *T. creatinini* zu kodieren, dem drittem Enzym des Creatininabbauweges.

### Beispiel 3: Heterologe Expression und Aufreinigung einer aktiven Creatinin-Deiminase aus *T. creatinini* in *E. coli*

Die heterologe Expression von aktiver Creatinin-Deiminase aus *T. creatinini* in *E. coli* wurde in einer Analyse des rekombinanten Zellextraktes mittels nativer Gelelektrophorese (Abb. 8) und Aktivitätsbestimmung im zellfreien Rohextrakt nachgewiesen. Die native Gelelektrophorese zeigte, dass im Falle von *E. coli* DH5$\alpha$/pKT1 im Gegensatz zu *E. coli* DH5$\alpha$/pKS$^+$ eine zusätzliche eindeutige Bande im Bereich von 300 kDa auftrat (vgl. Abb. 8, Spur 4 und Spur 6). Auf gleicher Höhe befand sich die Bande der gereinigten Creatinin-Deiminase (Abb. 8, Spur 3 und 5), die sich ebenfalls im Rohextrakt von *T. creatinini* zeigte (Abb. 8, Spur 2). Die Größe lag damit in dem Bereich (288 kDa), der von Gottschalk et al. 1991 (supra) ermittelt wurde. Die spezifische Aktivität der rekombinanten Creatinin-Deiminase im zellfreien Extrakt lag bei ca. 10,2 U/mg.

Die heterolog exprimierte Creatinin-Deiminase (EC 3.5.4.21) aus *T. creatinini* wurde gemäß Gottschalk et al. 1991 (supra) aufgereinigt. Die Creatinin-Deiminase wurde jeweils aus 5 g Zellen mittels Ammoniumsulfatpräzipitation und Chromatographie an Phenylsepharose und DEAE-SepharoseCL6B zur apparenten Homogenität gereinigt (vgl. Abb. 9). Die spezifische Aktivität des gereinigten rekombinanten Enzyms wurde mit 1423 U/mg bestimmt (vgl. Tab. 2). Die Lagerung erfolgte bei -20°C in Kaliumphosphatpuffer (KPP; pH 7,65, 50 % Glycerin). Im folgenden werden die einzelnen Schritte der Reinigung beschrieben.

### Stufe 1: Herstellung des zellfreien Extraktes

5 g Zellmasse wurden in 0,05 mol/l Kaliumphosphat, pH 7,65 unter Zusatz von 0,1 mg/ml Ribonuklease und 0,1 mg/ml Desoxyribonuclease resuspendiert und durch dreimalige Behandlung mit einer French-Presse unter einem Druck von 120 MPa weitgehend aufgeschlossen. Zellrückstände wurden durch Zentrifugation (50000 x g, 10 min und 4°C) entfernt. Der zellfreie Überstand wurde als Rohextrakt bezeichnet.

### Stufe 2: Ammoniumsulfatfällung

Der erhaltene Rohextrakt (14,7 ml) wurde auf eine 0,65 Sättigung mit Ammoniumsulfat bei 0°C gebracht. Es wurde abzentrifugiert (50000 x g, 15 min, 4°C) und das Präzipitat mit dem Enzym in 3 ml 0,05 mol/l Kaliumphosphatpuffer, pH 7,65 gelöst. Zur Vorbereitung der Phenyl-Sepharose High Performance Chromatographie wurde gegen 0,05 mol/l Kaliumphosphatpuffer, pH 7, der 1,7 mol/l $(NH_4)_2SO_4$ enthielt, dialysiert.

**Stufe 3: Phenyl-Sepharose High Performance Chromatographie**

Das dialysierte Enzym wurde an einer Phenyl-Sepharose High Performance-Säule chromatographiert, die mit einer Lösung von 0,05 mol/l Kaliumphosphat pH 7/1,7 mol/l $(NH_4)_2SO_4$ äquilibriert worden war. Das an die Säule gebundene Enzym wurde durch Verringerung der $(NH_4)_2SO_4$-Konzentration in dem Puffer mit einem absteigenden linearen Salzgradienten von 100 bis 0% $(NH_4)_2$ $SO_4$ eluiert. Fraktionen mit 2,75 ml wurden bei einer Durchflussrate von 1 ml/min gesammelt. Die Enzymaktivität eluierte bei 0,85 mol/l $(NH_4)_2SO_4$ in einem Gesamtvolumen von 55 ml. Diese wurden unter mehrmaligen Wechsel des Puffers (0,05 mol/l Kaliumphosphat, pH 7,65/Glycerin (1:2 vol/vol)) bei 4°C bis zur Ammoniumfreiheit dialysiert.

**Stufe 4: DEAE CL6B Chromatographie**

Das gesammelte dialysierte Enzym vom letzten Reinigungsschritt wurde an einer DEAE CL 6B-Säule (1 x 10 cm) mit einem aufsteigenden linearen Gradienten von 0,015 mol/l bis 1 mol/l KCl in 0,05 mol/l Kaliumphosphatpuffer, pH 7,65 chromatographiert. Das Enzym eluierte bei 0,44 mol/l KCl in einem Volumen von 4,5 ml. Diese Fraktion wurde wie in Schritt 3 beschrieben dialysiert. Das Reinigungsschema ist in Tabelle 2 beschrieben.

Das gereinigte Enzym war im Dialysepuffer über einen Zeitraum von 9 Monaten mit einem Aktivitätsverlust von 9,1 % haltbar. Der optimale pH-Wert für das gereinigte Enzym lag in Phosphat- und TEA-Puffem im Bereich von 8,5 - 8,75, die maximale Aktivität wurde bei 47°C gemessen.

**Tabelle 2: Reinigung der rekombinanten Creatinin-Deiminase** *In Gegen wart von Ammoniumsulfat (Stufe 1-4) wurde die Enzymaktivität durch die Jaffé-Methode bestimmt, in Stufe 5 durch den gekoppelten optischen Test.

| Reinigungsstufe | Volumen (ml) | Aktivität * (U/ml) | Protein (mg/ml) | Spez. Aktivität (U/mg) |
|---|---|---|---|---|
| 1) Rohextrakt | 14,7 | 265 | 26 | 10,2 |
| 2) Ammoniumsulfat 65% Überstand | 23 | 2,2 | 6,7 | 0,33 |
| 3) Ammoniumsulfat 65 % resuspendiertes Pellet | 3 | 981 | 1,81 | 542 |
| 4) Phenyl-Sepharose HP | 7,2 | 195 | 0,19 | 1026 |
| 5) DEAE-Sepharose CL6B | 1,5 | 569 | 0,4 | 1423 |

**Beispiel 4: Charakterisierung der gereinigten rekombinanten Creatinin-Deiminase**

Homogenität und Molare Masse

Das aufgereinigte rekombinante Enzym wanderte als eine Einzelbande in einem nativen PhastGel-Gradienten (8-25%) zwischen den Referenzproteinen Ferritin (440 kDa) und Katalase (232 kDa). Dieses zeigte die Homogenität der gereinigten Creatinin-Deiminase nach den Kriterien des nativen PhastGel-Gradienten. Die relative molekulare Masse des Enzyms wurde durch Extrapolation mittels der Kalibratoren mit 296 kDa berechnet (Abb. 9, Spur 2 und 3). Um die Größe der Polypeptidketten der Creatinin Deiminase zu untersuchen, wurde eine SDS PhastGel-Gradient (10-15%) Elektrophorese mit der gereinigten Enzympräparation durchgeführt. Die elektrophoretische Mobilität der Bande entsprach einer relativen molekularen Masse von 49 kDa (Abb. 9, Spur 4 und 5) und war mit dem Wert von 45,7 kDa vergleichbar, der aufgrund der abgeleiteten Aminosäuresequenz des *cdi*-Gens berechnet wurde.

Nach der Auftrennung des rekombinanten und des nativen Enzyms durch eine SDS/Polyacrylamid (12%) Gelelektrophorese konnte mit Hilfe eines Westernblots gezeigt werden, dass Antikörper gegen das rekombinante Enzym sowohl mit diesem als auch mit dem nativen Enzym aus *Tissierilla creatinini* reagierten (Abb. 10, Spur 5-7, bzw. Spur 1-3).

Katalytische Eigenschaften und Metallgehalt der rekombinant in *E. coli* hergestellten Creatinin Deiminase

Die Substratsättigungskurve des gereinigten Enzyms für Creatinin ist in Abb. 11 gezeigt. Der $K_m$-Wert für Creatinin wurde basierend auf einer Auftragung nach Lineweaver Burk mit 1,1 mmol/l berechnet, der $V_{max}$ für diese Enzympräparation mit 8,8 U/ml (Proteinkonzentration: 6 µg/ml --> 1467 U/mg).

Eine Metallanalyse der aufgereinigten rekombinanten Creatinin-Deiminase ergab einen Gehalt von 1,8 Zinkatomen pro 47,5 kDa Untereinheit.

**Beispiel 5: Enzymtest zur Bestimmung von Creatinin**

Die Aktivität des gereinigten rekombinanten Enzyms wurde durch eine modifizierte enzymatische Methode nach Bergmeyer 1985 (BERGMEYER, H.U. (1985). Methods of enzymatic analysis 8: 488-507) bestimmt. Sie beruht auf der Messung des während der Creatinindeiminierung freigesetzten Ammoniaks in einem gekoppelten optischen Test durch Verbrauch von NADH bei 340 nm bzw. 365 nm. Der Reaktionsansatz enthielt folgende Konzentrationen in einem Endvolumen von 0,6 ml: TEA-Puffer 0,11 mol/l, pH 8,6; Creatinin 1,5 mmol/l; ADP 1,11 mmol/l; DTE 1 mmol/l; NADH 0,11 mmol/l; 2-Oxoglutarat 11 mmol/l; Glutamat-Dehydrogenase 8 U/ml. Nach Inkubation des Reaktionsansatzes (5 min bei 37°C) in einer Küvette im Photometer wurde die Reaktion mit 0,01 ml des gereinigten rekombinanten Enzyms gestartet und die Extinktionsabnahme bei 340 nm beobachtet. Eine Enzymeinheit katalysiert den Abbau von 1 µmol Creatinin/min bei 37°C.

**Beispiel 6: Creatininbestimmungen im Plasma, Serum und Urin**

Die Bestimmung der Creatininmenge erfolgte in Übereinstimmung mit dem in Beispiel 5 beschriebenen Testansatz. Der benutzte Probenleerwert enthielt alle Reagenzien ausgenommen das Enzym. Die Creatininkonzentration wurde wie folgt berechnet:

$$C_{Creatinin} = \frac{\Delta E \times Mw \times V}{\varepsilon_{340} \times d \times v \times 10}$$

V = Endvolumen (ml); v = Probenvolumen (ml); Mw = Molekulargewicht des Creatinins; $\varepsilon_{340}$= spezifischer mikromolarer Extinktionskoeffizient von NADH (6,3 $cm^2$/µmol); d = Lichtweg (cm)

Es wurden Interferenzstudien mit der gereinigten heterolog exprimierten Creatinin-Deiminase mit verschiedenen in Seren relevanten Substanzen durchgeführt. Diese wurden zum Standardtestansatz gegeben und jeweils die Aktivität der Creatinin-Deiminase bestimmt. In Tabelle 1 sind die getesteten relevanten Substanzen aufgelistet, die in den angegebenen Konzentrationen keine Interferenzen mit dem Enzymtest zeigten. Die rekombinante Creatinin-Deiminase zeigte eine außerordentliche Substratspezifität bei Zusatz der in Tabelle 1 angegebenen Substanzen.

**Beispiel 7: Einsatz in Routineuntersuchungen**

Das vorstehend beschriebene Verfahren der Creatinin-Bestimmung im optischen Test wurde in der Routine angewendet und mit der enzymatischen Creatinin-Bestimmung von Roche-Diagnostik (Testprinzip vgl. Abb. 3) verglichen. Hierzu wurden beide Messmethoden unter Verwendung eines Hitachi 717-Gerätes durchgeführt, das mittels käuflicher Standards kalibriert worden war. Der Vergleich, der in Abb. 12 dargestellt ist, zeigt, dass der gekoppelte optische Test unter Verwendung der Creatinin-Deiminase den Anforderungen einer Routineuntersuchung genügt.

## Patentansprüche

1. Nukleinsäuresequenz, die ausgewählt ist aus:

    (i) einer Nukleinsäuresequenz, die für ein Protein oder Polypeptid mit Creatinin-Deiminase-Aktivität kodiert, ausgewählt aus der Gruppe, bestehend aus:

        (a) der in SEQ ID NO: 1 gezeigten Nukleinsäuresequenz oder einem Teil oder Derivat davon,
        (b) einer Nukleinsäuresequenz, die mit der Nukleinsäuresequenz gemäß (a) hybridisiert und/oder mindestens 50% Homologie zu dieser aufweist,
        (c) einer Nukleinsäuresequenz, die bezogen auf eine Nukleinsäuresequenz gemäß (a) oder (b) degeneriert ist,

    und
    (ii) einer Nukleinsäuresequenz, die zu der Nukleinsäuresequenz gemäß (a), (b) oder (c) komplementär ist.

2. Nukleinsäuresequenz gemäß Anspruch 1, **dadurch gekennzeichnet, dass** das Protein oder Polypeptid mit Creatinin-Deiminase-Aktivität Cytosin nicht deaminiert.

3. Nukleinsäuresequenz gemäß Anspruch 1 oder 2, **dadurch gekennzeichnet, dass** die Nukleinsäuresequenz aus *Tissierella creatinini* abgeleitet ist.

4. Nukleinsäuresequenz gemäß einem der Ansprüche 1 bis 3, **dadurch gekennzeichnet, dass** die Nukleinsäuresequenz DNA oder RNA ist.

5. Rekombinantes DNA-Molekül, das eine Nukleinsäuresequenz gemäß einem der Ansprüche 1 bis 4 umfasst und gegebenenfalls ferner (i) Expressionskontrollsequenzen, die die Expression der Nukleinsäuresequenz steuern, und vorzugsweise einen Promotor umfassen, und/oder (ii) eine Nukleinsäuresequenz, die für ein Polypeptid kodiert, das eine Sekretion des durch die Nukleinsäuresequenz kodierten Proteins oder Polypeptids steuert, umfasst, wobei das rekombinante DNA-Molekül vorzugsweise ein Vektor oder Plasmid, insbesondere ein viraler Vektor oder ein Bakteriophage ist.

6. Rekombinantes DNA-Molekül gemäß Anspruch 5, **dadurch gekennzeichnet, dass** die Expressionskontrollsequenzen homo- oder heterolog zu der Nukleinsäuresequenz sind.

7. Rekombinantes DNA-Molekül gemäß Anspruch 5 oder 6, **dadurch gekennzeichnet, dass** die Expression regulierbar ist.

**8.** Wirtszelle, die ein DNA-Molekül gemäß einem der Ansprüche 5 bis 7 umfasst, wobei die Wirtszelle vorzugsweise eine prokaryotische Zelle, insbesondere eine prokaryotische Zelle, die aus *Escherichia coli* und *Bacillus subtilis* ausgewählt ist, eine Hefezelle, eine Insektenzelle, eine Pflanzenzelle oder eine Säugerzelle ist.

**9.** Wirtszelle gemäß Anspruch 8, **dadurch gekennzeichnet, dass** die Nukleinsäuresequenz in der Wirtszelle exprimiert und gegebenenfalls das durch die Nukleinsäuresequenz kodierte Protein oder Polypeptid sekretiert wird.

**10.** Protein oder Polypeptid, das ausgewählt ist aus (i) einem Protein oder Polypeptid, das durch eine Nukleinsäuresequenz gemäß einem der Ansprüche 1 bis 3 kodiert wird, und (ii) einem Protein oder Polypeptid mit Creatinin-Deiminase-Aktivität, das die in SEQ ID NO:2 gezeigte Aminosäuresequenz, einen Teil oder ein Derivat davon umfasst.

**11.** Antikörper, insbesondere monoklonaler Antikörper, der spezifisch mit einem Protein oder Polypeptid gemäß Anspruch 10 reagiert.

**12.** Verfahren zur Herstellung eines Proteins oder Polypeptides mit Creatinin-Deiminase-Aktivität, **dadurch gekennzeichnet, dass** eine Nukleinsäuresequenz gemäß einem der Ansprüche 1 bis 4 in einer Wirtszelle exprimiert und das Protein oder Polypeptid isoliert wird, wobei die Isolierung des Proteins oder Polypeptids vorzugsweise durch Ammoniumsulfatfällung und Chromatographie an Phenylsepharose und DEAE-SepharoseCL6B erfolgt.

**13.** Verfahren zur Bestimmung der Creatinin-Konzentration in einer Probe, **dadurch gekennzeichnet, dass** das Verfahren die Schritte umfasst:

(a) Reaktion des Creatinins mit einem Protein oder Polypeptid gemäß Anspruch 10,
(b) Bestimmung der Menge des in Schritt (a) gebildeten Ammoniaks, wobei die Probe vorzugsweise eine Körperflüssigkeit, insbesondere Plasma, Serum oder Urin, enthält.

**14.** Verfahren gemäß Anspruch 13, **dadurch gekennzeichnet, dass** die Bestimmung des Ammoniaks durch Behandlung mit Glutamatdehydrogenase in Gegenwart von $\alpha$-Ketoglutarat und NADH oder NADPH und Messung des Verbrauchs an NADH oder NADPH erfolgt, wobei die Messung des Verbrauchs an NADH oder NADPH vorzugsweise durch photometrische Messung bei 340 bzw. 365 nm erfolgt.

**15.** Kit zur Bestimmung der Creatinin-Konzentration, umfassend

(a) eine Nukleinsäuresequenz gemäß einem der Ansprüche 1 bis 4 oder
(b) eine Wirtszelle gemäß Anspruch 8 oder 9 oder
(c) ein Protein oder Polypeptid gemäß Anspruch 10.

**16.** Verwendung einer Nukleinsäuresequenz gemäß einem der Ansprüche 1 bis 4 zur Herstellung eines Proteins oder Polypeptids mit Creatinin-Deiminase-Aktivität.

**17.** Verwendung eines Proteins oder Polypeptids gemäß Anspruch 10 zur Bestimmung der Creatinin-Konzentration in einer Probe.

## Abb. 1

**In der Niere:**

L-Arginin + Glycin —Transamidase→ Ornithin + **Guanidinacetat**

**In der Leber:**

S-Adenosylmethionin + **Guanidinacetat** —Guanidinoacetat-Methyltransferase→ **Creatin** + S-Adenosylhomocystein

**Im Muskel:**

**Creatin** + ATP ⇄ (Creatinkinase) **Creatinphosphat** + ADP

**Creatinphosphat** → **Creatinin** + Phosphat

## Abb. 2

$$\text{Creatinin} + H_2O \xrightarrow{\text{Creatininase}} \text{Creatin}$$

$$\text{Creatin} + \text{ATP} \xrightarrow{\text{Creatin-Kinase}} \text{Creatinphosphat} + \text{ADP}$$

$$\text{ADP} + \text{Phosphoenolpyruvat} \xrightarrow{\text{Pyruvat-Kinase}} \text{ATP} + \text{Pyruvat}$$

$$\text{Pyruvat} + \text{NADH}+H^+ \xrightarrow{\text{Lactatdehydrogenase}} \text{Lactat} + \text{NAD}^+$$

# Abb. 3

Creatinin + $H_2O$ $\xrightarrow{\text{Creatininase}}$ Creatin

Creatin + $H_2O$ $\xrightarrow{\text{Creatinase}}$ Harnstoff + Sarcosin

Sarcosin + $O_2$ + $H_2O$ $\xrightarrow{\text{Sarcosinoxidase}}$ Glycin + Formaldehyd + $H_2O_2$

$H_2O_2$ + Indikator (PAP) $\xrightarrow{\text{Peroxidase}}$ 2 $H_2O$ + HBr + Farbstoff

**Abb. 4**

$$\text{Creatinin} \xrightarrow{\text{Creatinin-Deiminase}} \text{N-Methylhydantoin} + NH_3$$

$$NH_3 + \alpha\text{-Ketoglutarat} + \text{NADPH+H}^{\oplus} \xrightarrow{\text{Glutamatdehydrogenase}} \text{Glutamat} + \text{NADP}^{+}$$

**Abb. 5**

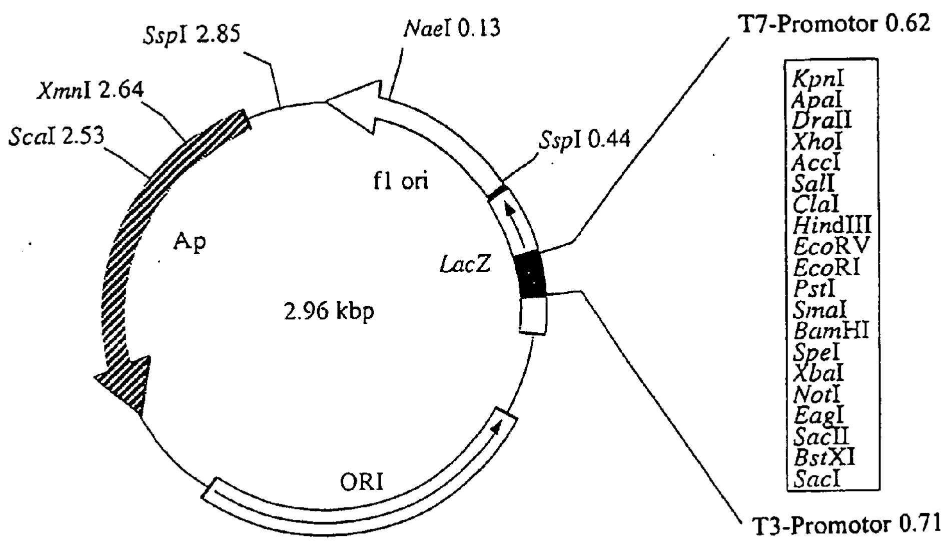
SspI 2.85
NaeI 0.13
T7-Promotor 0.62
XmnI 2.64
ScaI 2.53
SspI 0.44
f1 ori
Ap
LacZ
2.96 kbp
ORI
KpnI
ApaI
DraII
XhoI
AccI
SalI
ClaI
HindIII
EcoRV
EcoRI
PstI
SmaI
BamHI
SpeI
XbaI
NotI
EagI
SacII
BstXI
SacI
T3-Promotor 0.71

**Abb. 6**

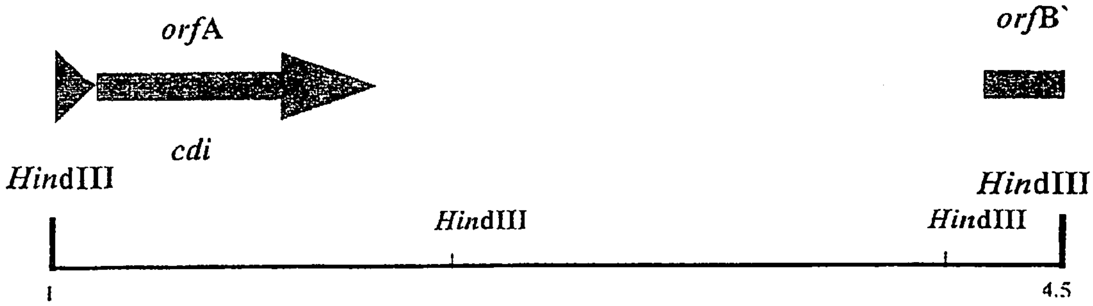


pKT1 (insert 4.5 kbp)

# Abb. 7

```
CTGGCATTAGTGTTATTGGCTATAGCAACAATTTTGTCAATAACTGATAAAAATACATTAACAAAAGAAAAACTGTAAGC   80
                    rbs
TATTAACAATGCTAAATTTTTAAGGAGTGATTTTATGATGAAAAAGTTTATTAATGCAAAGATTTACAAGAACAATGAAG  160
                                     M  M  K  K  F  I  N  A  K  I  Y  K  N  N  E  A   16
                                     ------ cdi ----->
CAACAGAAATTTTAGTAGAAGACGGTAAAATCAAAGAGATTGGTAATAACTTAGCAGACTGTAAAGAAGTAATTGATCTA  240
 T  E  I  L  V  E  D  G  K  I  K  E  I  G  N  N  L  A  D  C  K  E  V  I  D  L    42

GGCGGTAAAATGGTTACTCCACCTTATGTAGATCCTCACCTACATTTAGATTATGTGTATACATTGGCTGAACTTGGAAA  320
G  G  K  M  V  T  P  P  Y  V  D  P  H  L  H  L  D  Y  V  Y  T  L  A  E  L  G  K    69

AACTGGTGCTGGCTCAGGAACTCTTTTTGAAGCTATTGAAATGTGGCCAGTATTTAAAAAGACTTTAACTGTAGAAAGCG  400
 T  G  A  G  S  G  T  L  F  E  A  I  E  M  W  P  V  F  K  K  T  L  T  V  E  S  V   96

TTAAAAAACTTGCTCTTAAGGGGGTTATGGATGAGGTTTCCCAAGGGGTACAACATATTCGTACACATATAGATGTAACT  480
 K  K  L  A  L  K  G  V  M  D  E  V  S  Q  G  V  Q  H  I  R  T  H  I  D  V  T   122

GATCCAAAATTCACAGGTCTAAAAGCTATGTTGGAAATGAAAGAAGAATTAAAGGACATAGTTGATATCCAAATAGTATC  560
D  P  K  F  T  G  L  K  A  M  L  E  M  K  E  E  L  K  D  I  V  D  I  Q  I  V  S   149

ATTCCCACAACAAGGAATGTACACATATAAGGGTGGACGTGAATTAGTAGAAGAAGCACTTAAGATGGGTGCAGATGTTG  640
 F  P  Q  Q  G  M  Y  T  Y  K  G  G  R  E  L  V  E  E  A  L  K  M  G  A  D  V  V  176

TTGGAGGAATTCCGCATTATGAACCAGCTAGAGAATATGGTGAAATGTCTGTTAAAGCCACAGTTGAACTTGCTATGAAA  720
 G  G  I  P  H  Y  E  P  A  R  E  Y  G  E  M  S  V  K  A  T  V  E  L  A  M  K   202

TATGATAAGCTAATAGATGTTCACTGTGATGAGACAGATGATCCTCAAGCACGTTTTATTGAGCTATTAAATGCACTTGT  800
Y  D  K  L  I  D  V  H  C  D  E  T  D  D  P  Q  A  R  F  I  E  L  L  N  A  L  V   229

TTATTTGGAAGGTTATGGTGCAAAAACTTCAGCTAGCCATACTTGTTCATTTGGTTCAGCAGATGATTCATATGCATATA  880
 Y  L  E  G  Y  G  A  K  T  S  A  S  H  T  C  S  F  G  S  A  D  D  S  Y  A  Y  R  256

GAATGATAGACTTATTCAAAAAGAGCAAGATAAACTTCATCTCTAATCCAACTGAAAATGCGTATCTACAAGGCCGTCAT  960
 M  I  D  L  F  K  K  S  K  I  N  F  I  S  N  P  T  E  N  A  Y  L  Q  G  R  H   282

GACACTTATCCAAAGCGTCGTGGATTGACTAGAGTTAAAGAATTTATGGAGCATGGTATTAATGTTGCATTTGCACAAGA 1040
D  T  Y  P  K  R  R  G  L  T  R  V  K  E  F  M  E  H  G  I  N  V  A  F  A  Q  D   309

TTCAATAAACGATCCATGGTATCCAATGGGTAACGGAAATATGATGAATATACTTGACAATGGAATTCATTTAGCTCAAA 1120
S  I  N  D  P  W  Y  P  M  G  N  G  N  M  M  N  I  L  D  N  G  I  H  L  A  Q  I   336

TAATGTCACCACAAGATATAGAAAAAGATTTAGATTTAATTACCTACAATGGTGCTCGTTGCCTAAATATCCAAGATAAA 1200
 M  S  P  Q  D  I  E  K  D  L  D  L  I  T  Y  N  G  A  R  C  L  N  I  Q  D  K   362

TATTTATTAGAAGTAGGTAAAGATTCAAACTTTATCGTTCTTAACGGAGACAGCCCATTCGATGTAATAAGAAACCGTGC 1280
Y  L  L  E  V  G  K  D  S  N  F  I  V  L  N  G  D  S  P  F  D  V  I  R  N  R  A   389

TAATGTTCTTGCTTGTGTTAGAAAAGGAGAATTCTATTTAAGCAAAAACCAGTAGAATATGATGTAAAACTTGATTTAGG 1360
 N  V  L  A  C  V  R  K  G  E  F  Y  L  S  K  N  Q  *                           406
                                           term              term
CGTAAAATATTAATATTTTAAAATAAATTCCAAATTAACCCCCCGGTGGTGTAATAAACTCCATCGGGGGGTTTTTGTG 1440

TTCCAGTAGAAAATAAAAAAATGATATAAAAATTTAGTAGTTTGAAAAACTTAAATAAAGAAAGGGCGGATTTAGAATGA 1520

GTCAAAGAGACGTATTATATTCACCAGATGCAAAGTACAAAGATAATAAGGGTAAATATGGAATTGATTTAGG        1593
```

Abb. 8

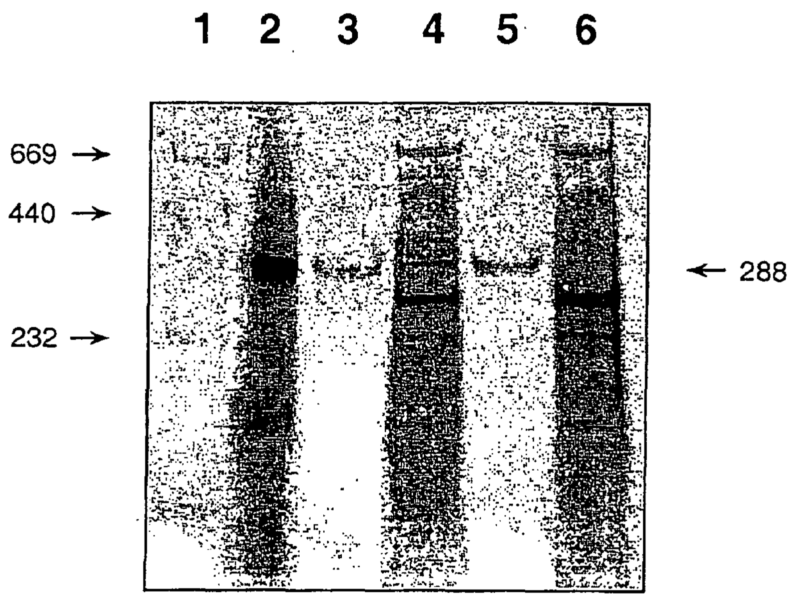

Abb. 9

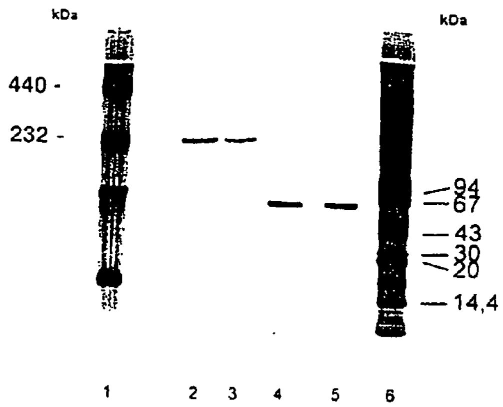

Abb. 10

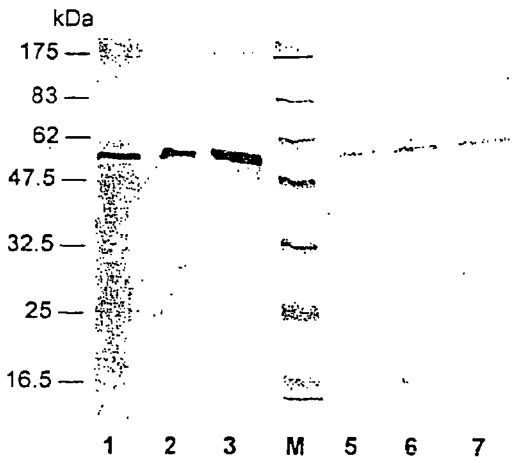

Abb. 11

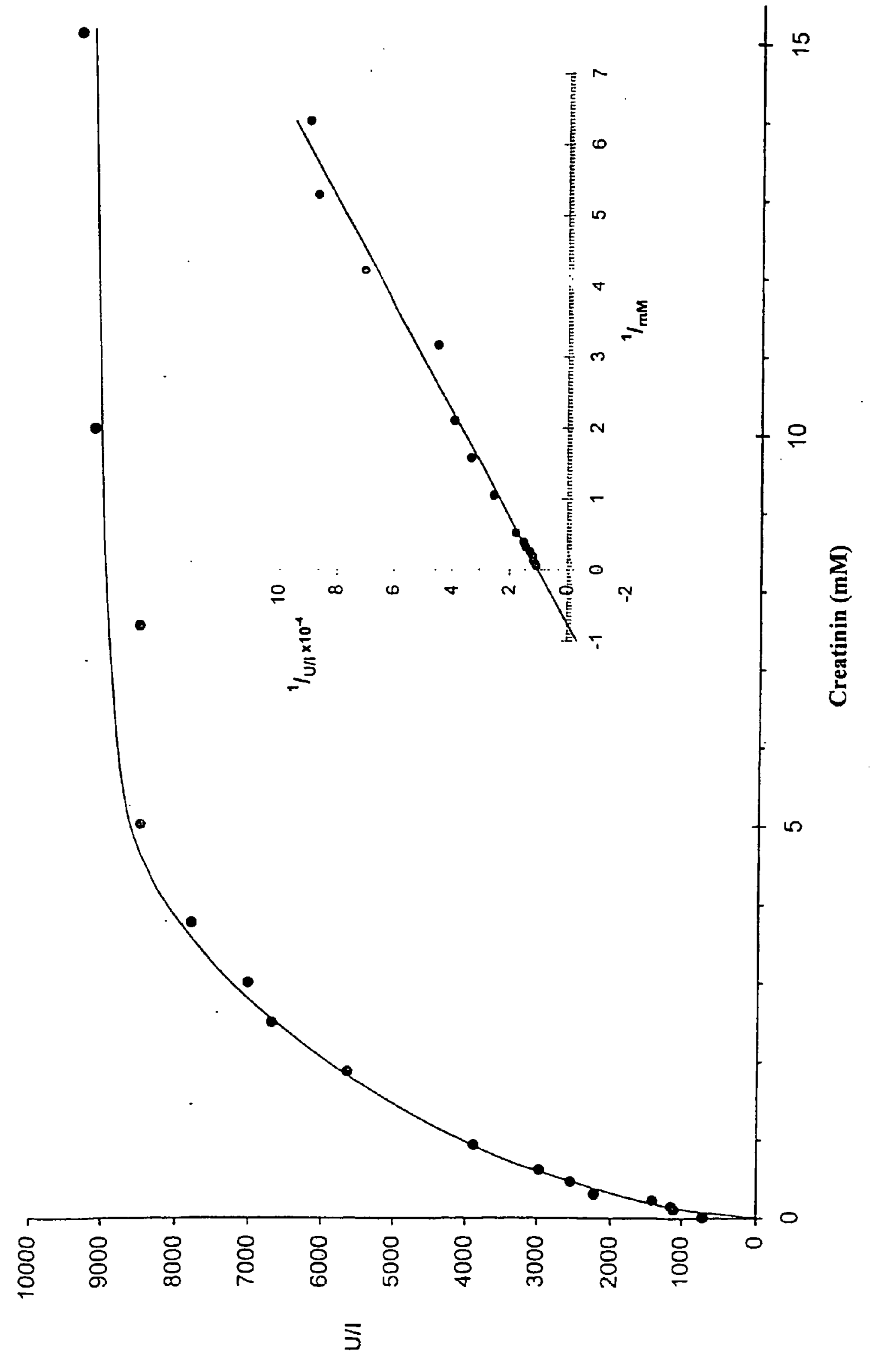

**Abb. 12**

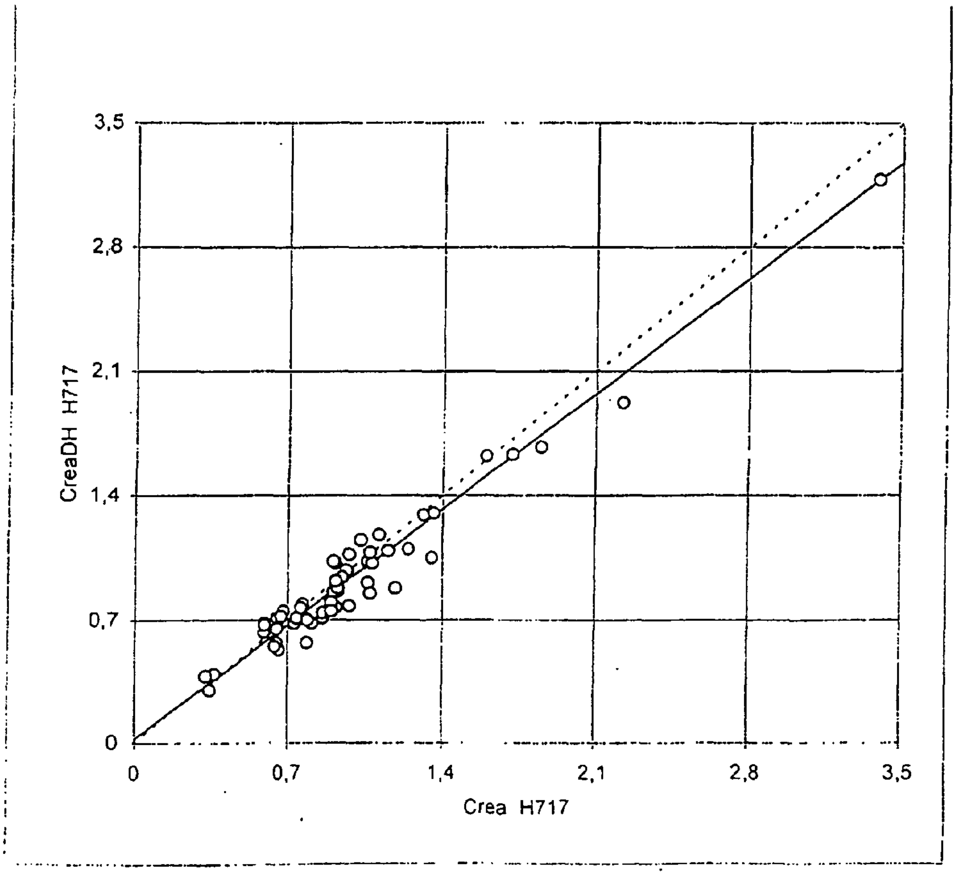

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **NARAYAMAN, S. ; APPLETON, H.D.** *Clin. Chem.,* 1980, vol. 26, 1119-1125
- **JAFFÉ, M.** *Z. Physiol. Chem.,* vol. 10, 391-400
- **SOLDIN, S.J. ; Henderson, L. ; Hill, J.G.** *Clin. Chem.,* 1978, vol. 26, 286-290
- **KROLL, M.H. ; HAGENGRUBER, C. ; ELIN, R.J.** *J. Biol. Chem.,* 1985, vol. 115, 333-341
- **SWAIN, R.R ; BRIGGS, S.L.** *Clin. Chem.,* 1977, vol. 23, 1340-1342
- **SAAH, A.J. ; KOCH, T.R. ; DRUSANO, G.L.** *JAMA,* 1982, vol. 247, 205
- **KNOLL, E. ; STAMM, D.** *J. Clin. Chem. Clin. Biochem.,* 1970, vol. 8, 582-587
- **KNOLL, E. ; WISSER, H.** *Z. Klin. Chem. Klin. Biochem.,* 1973, vol. 11, 411
- **POPPER, H. ; MANDEL, E. ; MAYER, H.** *Biochem. Z.,* 1969, vol. 291, 394
- **SCHEUERBRANDT, G. ; HELGER, R.** *Ärztl. Lab.,* 1969, vol. 15, 65
- **Van PILSUM, J.F. ; MARTIN, R.P. ; KITO, E. HESS, J.** *J. Biol. Chem.,* 1956, vol. 222, 225-236
- **LANGLEY, W.D. ; EVENS, M.** *J. Biol. Chem.,* 1936, vol. 115, 333-341
- **BENEDICT, S.R. ; BEHRE, J.A.** *J. Biol. Chem.,* 1936, vol. 114, 515-532
- **SIROTA, J.H ; BALDWIN, D.S. ; VILLAREAL, H.** *J. Clin. Invest.,* 1950, vol. 29, 187-192
- **GUDER, W.G. ; HOFFMANN, G.E. ; POPPE, W.A. ; SIEDEL, J. ; PRICE, C.P.** *J. Chem. Clin. Biochem.,* 1986, vol. 24, 889-902
- **Messreaktion, SZULMAJSTER, J.** *J. Bacteriol.,* 1958, vol. 75, 633-639
- **SHIREY, T.L.** *Clin. Biochem.,* 1983, vol. 16, 147-152
- **LIM, F.** *Clin. Chem.,* 1974, vol. 20, 871
- **TANGANELLI, E. ; PRINCIPE, L. ; BASSI, D. ; CAMBIAGHI, S. ; MURADOR, E.** *Clin. Chem.,* 1982, vol. 28, 1461-1464
- **MERRIFIELD, R.B. ; STEWART J.M.** Solid Phase Synthesis. *Nature,* 1965, vol. 207, 522-523
- **BRYANT, M. P.** *Am. J. Clin. Nutr.,* 1972, vol. 25, 1324-1328
- **HUNGATE, R. E.** Methods in Microbiology. Academic press, 1969, vol. 3B, 117-132
- **GAUGLITZ, U.** *Dissertation,* 1988
- **GOTTSCHALK, E. M. ; HIPPE, H. ; PATZKE, F.** *Clin. Chim. Acta,* 1991, vol. 204, 223-238
- **CLEVELAND, D. W. ; FISCHER, S. G. ; KIRSCHNER, M. W. ; LAEMMLI, U. K.** *J. Biol. Chem.,* 1977, vol. 252, 1102-1106
- **BERTRAM, J. ; DÜRRE, P.** *Arch. Microbiol.,* 1989, vol. 151, 551-557
- **HANAHAN, D.** *J. Mol. Biol.,* 1983, vol. 166, 557-580
- **STRAUSS, E.C. ; KOBORI, J.A. ; SIU, G. ; HOOD, L.E.** *Anal. Biochem.,* 1986, vol. 154, 353-360
- **MARCK, C.** *Nucl. Acids Res.,* 1988, vol. 16, 1829-1836
- **BERGMEYER, H.U.** *Methods of enzymatic analysis,* 1985, vol. 8, 488-507

SEQUENZPROTOKOLL

<110> CampusGen GmbH

<120> Genetische Sequenz, die für Creatinin-Deiminase kodiert und deren Verwendung

<130> 337-1 EPT1

<140>
<141>

<150> DE 102 00 386.6
<151> 08.01.2002

<160> 2

<170> PatentIn Ver. 2.1

<210> 1
<211> 1593
<212> DNA
<213> Tissierella creatinini

<400> 1
ctggcattag tgttattggc tatagcaaca attttgtcaa taactgataa aaatacatta 60
acaaaagaaa aactgtaagc tattaacaat gctaaatttt taaggagtga ttttatgatg 120
aaaaagttta ttaatgcaaa gatttacaag aacaatgaag caacagaaat tttagtagaa 180
gacggtaaaa tcaaagagat tggtaataac ttagcagact gtaaagaagt aattgatcta 240
ggcggtaaaa tggttactcc accttatgta gatcctcacc tacatttaga ttatgtgtat 300
acattggctg aacttggaaa aactggtgct ggctcaggaa ctctttttga agctattgaa 360
atgtggccag tatttaaaaa gactttaact gtagaaagcg ttaaaaaact tgctcttaag 420
ggggttatgg atgaggtttc ccaaggggta caacatattc gtacacatat agatgtaact 480
gatccaaaat tcacaggtct aaaagctatg ttggaaatga aagaagaatt aaaggacata 540
gttgatatcc aaatagtatc attcccacaa caaggaatgt acacatataa gggtggacgt 600
gaattagtag aagaagcact taagatgggt gcagatgttg ttggaggaat tccgcattat 660
gaaccagcta gagaatatgg tgaaatgtct gttaaagcca cagttgaact tgctatgaaa 720
tatgataagc taatagatgt tcactgtgat gagacagatg atcctcaagc acgttttatt 780
gagctattaa atgcacttgt ttatttggaa ggttatggtg caaaaacttc agctagccat 840
acttgttcat ttggttcagc agatgattca tatgcatata gaatgataga cttattcaaa 900
aagagcaaga taaacttcat ctctaatcca actgaaaatg cgtatctaca aggccgtcat 960
gacacttatc caaagcgtcg tggattgact agagttaaag aatttatgga gcatggtatt 1020
aatgttgcat ttgcacaaga ttcaataaac gatccatggt atccaatggg taacggaaat 1080
atgatgaata tacttgacaa tggaattcat ttagctcaaa taatgtcacc acaagatata 1140
gaaaaagatt tagatttaat tacctacaat ggtgctcgtt gcctaaatat ccaagataaa 1200
tatttattag aagtaggtaa agattcaaac tttatcgttc ttaacggaga cagcccattc 1260
gatgtaataa gaaaccgtgc taatgttctt gcttgtgtta gaaaaggaga attctattta 1320
agcaaaaacc agtagaatat gatgtaaaac ttgatttagg cgtaaaatat taatatttta 1380
aaataaattc caaattaacc ccccggtggt gtaataaact ccatcggggg gtttttgtg 1440
ttccagtaga aaataaaaaa atgatataaa aatttagtag tttgaaaaac ttaaataaag 1500
aaagggcgga tttagaatga gtcaaagaga cgtattatat tcaccagatg caaagtacaa 1560
agataataag ggtaaatatg gaattgattt agg 1593

<210> 2
<211> 406
<212> PRT
<213> Tissierella creatinini

<400> 2

```
Met Met Lys Lys Phe Ile Asn Ala Lys Ile Tyr Lys Asn Asn Glu Ala
1               5                   10                  15

Thr Glu Ile Leu Val Glu Asp Gly Lys Ile Lys Glu Ile Gly Asn Asn
            20                  25                  30

Leu Ala Asp Cys Lys Glu Val Ile Asp Leu Gly Gly Lys Met Val Thr
        35                  40                  45

Pro Pro Tyr Val Asp Pro His Leu His Leu Asp Tyr Val Tyr Thr Leu
    50                  55                  60

Ala Glu Leu Gly Lys Thr Gly Ala Gly Ser Gly Thr Leu Phe Glu Ala
65                  70                  75                  80

Ile Glu Met Trp Pro Val Phe Lys Lys Thr Leu Thr Val Glu Ser Val
                85                  90                  95

Lys Lys Leu Ala Leu Lys Gly Val Met Asp Glu Val Ser Gln Gly Val
            100                 105                 110

Gln His Ile Arg Thr His Ile Asp Val Thr Asp Pro Lys Phe Thr Gly
        115                 120                 125

Leu Lys Ala Met Leu Glu Met Lys Glu Glu Leu Lys Asp Ile Val Asp
    130                 135                 140

Ile Gln Ile Val Ser Phe Pro Gln Gln Gly Met Tyr Thr Tyr Lys Gly
145                 150                 155                 160

Gly Arg Glu Leu Val Glu Glu Ala Leu Lys Met Gly Ala Asp Val Val
                165                 170                 175

Gly Gly Ile Pro His Tyr Glu Pro Ala Arg Glu Tyr Gly Glu Met Ser
            180                 185                 190

Val Lys Ala Thr Val Glu Leu Ala Met Lys Tyr Asp Lys Leu Ile Asp
        195                 200                 205

Val His Cys Asp Glu Thr Asp Asp Pro Gln Ala Arg Phe Ile Glu Leu
    210                 215                 220

Leu Asn Ala Leu Val Tyr Leu Glu Gly Tyr Gly Ala Lys Thr Ser Ala
225                 230                 235                 240

Ser His Thr Cys Ser Phe Gly Ser Ala Asp Asp Ser Tyr Ala Tyr Arg
                245                 250                 255

Met Ile Asp Leu Phe Lys Lys Ser Lys Ile Asn Phe Ile Ser Asn Pro
            260                 265                 270

Thr Glu Asn Ala Tyr Leu Gln Gly Arg His Asp Thr Tyr Pro Lys Arg
        275                 280                 285

Arg Gly Leu Thr Arg Val Lys Glu Phe Met Glu His Gly Ile Asn Val
    290                 295                 300

Ala Phe Ala Gln Asp Ser Ile Asn Asp Pro Trp Tyr Pro Met Gly Asn
305                 310                 315                 320
```

```
Gly Asn Met Met Asn Ile Leu Asp Asn Gly Ile His Leu Ala Gln Ile
                325                 330                 335

Met Ser Pro Gln Asp Ile Glu Lys Asp Leu Asp Leu Ile Thr Tyr Asn
            340                 345                 350

Gly Ala Arg Cys Leu Asn Ile Gln Asp Lys Tyr Leu Leu Glu Val Gly
        355                 360                 365

Lys Asp Ser Asn Phe Ile Val Leu Asn Gly Asp Ser Pro Phe Asp Val
    370                 375                 380

Ile Arg Asn Arg Ala Asn Val Leu Ala Cys Val Arg Lys Gly Glu Phe
385                 390                 395                 400

Tyr Leu Ser Lys Asn Gln
                405
```